# EUROPEAN PATENT APPLICATION

(11) **EP 1 661 588 A1**
(43) Date of publication of application: **31.05.2006**
(21) Application number: 04078231.0
(22) Date of filing: 29.11.2004
(51) Int. Cl.: A61L 9/12, A01M 1/20, B60H 3/00

(54) **Container for diffusion of volatile liquids**

(71) Applicant: Mannarino, Maria Teresa, 20020 Cogliate (Milano) (IT); Cipolla, Fiorenzo, 20020 Cogliate (Milano) (IT)
(72) Inventor: Mannarino, Maria Teresa, 20020 Cogliate (Milano) (IT); Cipolla, Fiorenzo, 20020 Cogliate (Milano) (IT)
(74) Representative: Riccardi, Sergio

(57) **Abstract**

A container for diffusion of volatile liquids is disclosed, comprising a bottle (1) containing said liquid (4), a cover (6) applied on the bottle, the top of the cover (6) meeting the bottom of a closure cap (9) screwed on the neck (2) of the bottle, characterized by a drawing tube (13) through which the liquid is delivered when said bottle is tilted or shaken.

## Description

The present invention relates to a container for diffusing volatile liquids having a significant utility and being user friendly.

Various designs of these containers are known, diffusing into the environment the contained liquid by impregnation of a stopper, a wick or a cord. The liquid may be a deodorant, a perfume or an essence possibly mixed with other substances such as moth-repellents, insect-repellents, insecticides and so forth.

However, the containers presently available on the market have some drawbacks, such as oozing of droplets on the surrounding objects and the difficulty of dosing the amount of liquid to be delivered. Other containers solve the problem with elements having the function of metering valve, thus adding to the cost and complexity of the article. Still other containers, hanging on the dashboard of a motor vehicle, may damage the glass of the windshield with repeated impacts when swinging back and forth.

The present invention brilliantly solves these problems, providing for a practical, strong container harmless for persons and objects, where the user may also carry out any desired dosage of the liquid to be delivered.

The container of the instant invention may be used either leaning on a plane or suspended by a string and therefore for vehicles and houses as well. Its features will be apparent from the following detailed description to be read with reference to the annexed sheets of illustrative drawings as indicated in the appended claims.

In the drawings:

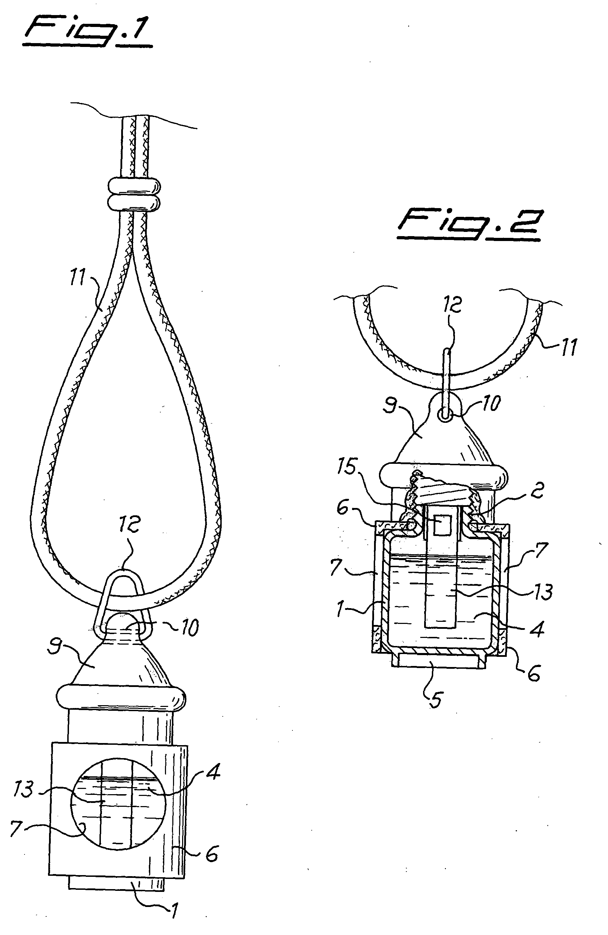

Fig. 1 is a front view of the container;
Fig. 2 is a similar cross-sectional view; and

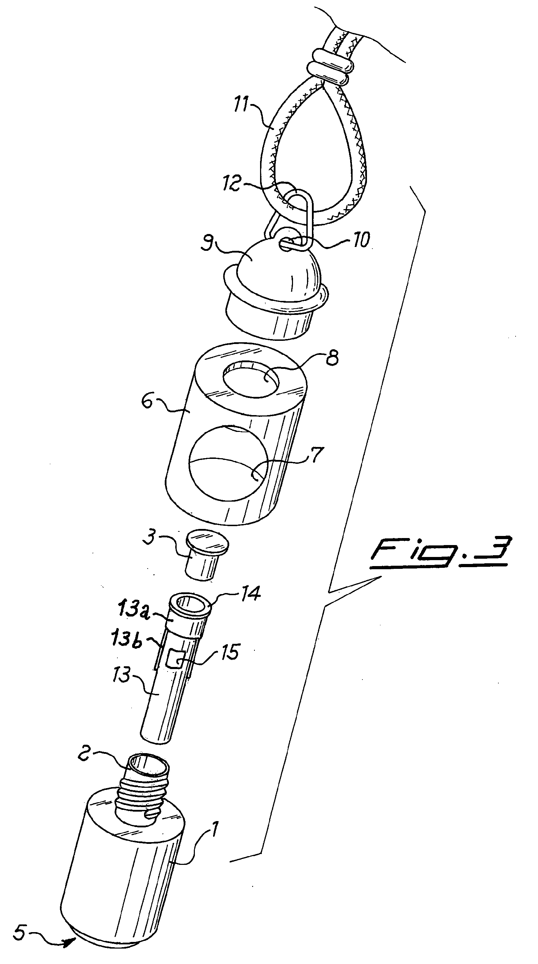

Fig. 3 is an exploded perspective view showing the container with its components to be assembled.

First of all it is to be noted that the embodiment shown in the drawings and described hereinafter relates to the suspended use, for instance on the driving mirror of a motor vehicle or the clothing hanging bar of a wardrobe, but the container is not limited to this use and it can also be placed on a plane, preferably removing the hanging string.

With reference now to the figures of the annexed drawings, the container according to the present invention comprises a small bottle **1** preferably made of glass or transparent plastics, having an externally threaded neck **2** and a stopper **3.**

The liquid **4** to be diffused into the environment is filled in the bottle **1,** the liquid being a deodorant, a perfume or other fluid possibly added with insect-repellents, moth-repellents and so forth.

The bottle **1** may also be provided with a seat **5** for its application on a support or with a piece of adhesive material to stick it on a plane.

A cover **6** is placed on the bottle **1.** Said cover **6** is provided with an open bottom and the top end has an opening **8** for passage of the neck **2** of the bottle **1,** that is also provided with openings **7** allowing to check the level of liquid contained in the bottle, both when such level is high and is low, close to its exhaustion.

One can see that the cover **6** does not extend to the bottom of the bottle, to avoid that the delivered liquid stains or fouls the support plane when the bottle is used at home.

An internally threaded closure cap **9** is screwed on the threaded neck **2** of the bottle, so that the cap bottom meets the cover top.

At the top of the closure cap **9** there is a through hole **10** to apply an eyelet **12** for passage of a string **11** to be used for instance when the container must be suspended for instance to the driving mirror of a vehicle or inside a wardrobe. Moreover, the cover **6** avoids that the bottle **1** hits the windshield glass with consequent risk of damage.

It is to be noted that the string **11** has the only function of suspension element, because it has no direct contact with the closure cap **9,** cannot be impregnated with liquid, thus does not diffuse said liquid.

The container is also provided with a drawing tube **13** inserted into the bottle **1** until its rim **14** leans on the neck mouth **2.** The tube **13** closed by the stopper **3** allows to use and dose better the liquid of the bottle, up to the last droplet thanks to the slot **15** made on the tube at the connection point between the body and the neck of the bottle.

With this expedient the contents of the bottle may be easily dosed, has a longer duration and is fully delivered up to its complete exhaustion, as the slot **15** allows to collect even the last droplet of the liquid. Moreover, the pipe **13** reduces evaporation of the contents, reducing its consumption. Said pipe **13** may be made of any suitable material and preferably a non absorbent plastic material.

The drawing tube **13** has a stepped shape, with a first upper portion **13a** sealing the mouth of neck **2** of bottle **1,** a middle semicircular portion **13b** aiding the tube to slide when introduced into the neck **2,** and then the lower tube body depending in the liquid **4** contained in the bottle.

As already mentioned, the cover **6** and the closure cap **9** may be made of various materials. A preferred material is soft wood which can be easily impregnated by the liquid delivered by the bottle, but also several plastics may be used, whether or not adapted to be impregnated.

According to a further variation of the invention, cover and/or closure cap might be made or coated with a photocromic or thermocromic substance, absorbing the energy of the daylight and emitting it in the dark as luminescence, so that the container would be easily retrieved for instance inside the compartment of a vehicle.

Operation and use of the container of the present invention are clear.

The closure cap **9** is removed and the stopper **3** is removed. Then the closure cap is again screwed on the cover and the container is tilted or shaken shortly, so that a little quantity of liquid will be delivered and impregnate the cap **9** and the cover **6,** when they are made of a material that can be impregnated by the liquid such as soft wood.

When the delivered liquid ceased its effects, the operation should be repeated.

When the container is used in the suspended mode, it is sufficient to release it and the container will return by gravity to its vertical position stopping the delivery of further liquid.

The stopper **3** may also be finally removed and discarded, or it could be provided with a tear off or punching opening to further limit the liquid delivery, or with a little pad to be impregnated for liquid diffusion, if cover and cap are made of a non impregnated material.

It is to be pointed out again that shape and dimensions of the various components of the container are not influential as well as shape and dimensions of the openings made on the cover, and the choice of the most convenient materials for their manufacture as well, as defined in the appended claims.

## Claims

1. A container for diffusion of volatile liquids, comprising a bottle (1) containing said liquid (4), a cover (6) applied on the bottle, the top of the cover (6) meeting the bottom of a closure cap (9) screwed on the neck (2) of the bottle, **characterized by** a drawing tube (13) through which the liquid is delivered when said bottle is tilted or shaken.

2. The container of claim 1, **characterized in that** the drawing tube (13) is provided with a delivery slot (15) made at the connection point between body and neck of the bottle, so as to collect the liquid contents up to the last droplet by turning the container upside down.

3. The container according to claim 1 or 2, **characterized in that** the drawing tube (13) has a stepped shape (13a, 13b) sealing the mouth of the neck (2) of bottle (1).

4. The container of claim 1, **characterized in that** the cover (6) has one or more openings (7) to check the liquid level inside the bottle.

5. The container of claim 1, **characterized in that** the cover (6) does not extend to the bottom base of the bottle (1) not to foul the support plane when the bottle is placed on said plane.

6. The container according to claim 1, **characterized in that** the closure cap (9) has a through hole (10) at its top for application of an eyelet (12) for passage of a hanging string (11) for a suspended use of the container such as the driving mirror of a vehicle or inside a wardrobe.

7. The container according to claim 1, **characterized in that** the drawing tube (13) is provided with a removable stopper (3).

8. The container according to claim 1, **characterized in that** the bottle is provided with a tear-off or punching stopper.

9. The container according to claim 1, **characterized in that** the bottle bottom is provided with means (5) to fix the bottle on a support plane.

10. The container according to claim 1, **characterized in that** the closure cap (9) and/or the cover (6) are made of a wood adapted to be impregnated by the liquid (4).

11. The container according to claim 1, **characterized in that** the bottle (1) is made of glass or transparent plastics.

12. The container according to claim 1, **characterized in that** the cover (6) and/or the closure cap (9) are made of plastic material, possibly with means to make them luminescent in the dark.

13. The container according to claim 1, **characterized in that** the liquid (4) contained in the bottle is a deodorant, a perfume or other liquid, possibly mixed with insect-repellents, moth-repellents, insecticides and the like.
